# EUROPEAN PATENT APPLICATION

(11) **EP 2 387 937 A1**
(43) Date of publication of application: **23.11.2011**
(21) Application number: 11166457.9
(22) Date of filing: 17.05.2011
(51) Int. Cl.: A61B 1/00, A61B 1/005, A61B 1/07, A61B 1/313

(54) **Endoscope, particularly for spinal endoscopy**

(30) Priority: 17.05.2010 IT TO20100410
(71) Applicant: Forimpresit S.r.l., Alpignano (IT)
(72) Inventor: Forino, Luciano, 10100 Torino (IT)
(74) Representative: Jorio, Paolo

(57) **Abstract**

An endoscope (1) having a catheter (2), in turn having a flexible body (6) defining, along its longitudinal axis, at least an operating lumen (7) and an optical lumen (8), the body (6) having a distal end (5) insertable into an endoscopy target area; and an optical fibre bundle (9) housed in the optical lumen (8); the optical fibre bundle (9) being fixed firmly at one end (11) to the distal end (5) of the body (6) to permit direct visual access to the target area.

## Description

The present invention relates to an endoscope.

More specifically, the invention relates to an endoscope for performing spinal endoscopy procedures.

Spinal endoscopy, also known as epi- or periduroscopy, is an analgesic, low-invasion surgical method of directly accessing and in vivo displaying the peridural space hypodermically.

The procedure is normally performed using an endoscope comprising a flexible catheter, which, in addition to housing the actual endoscopy tool, defines an operating channel by which to administer drugs locally and perform diagnoses, biopsies and therapeutic procedures, such as lysing peridural adhesions, etc.

The actual endoscopy tool is normally a fiberscope, i.e. comprises a bundle of optical fibres, some for illuminating and others for acquiring real-time images of the target area.

Spinal endoscopy is commonly used in cases of peridural inflammation or adhesions, or other obstructions resulting directly or indirectly in pain to the back or legs. Though the cause of chronic backache is rarely easy to determine, it has very often been found to be associated with the abnormal presence of hypertrophic repair tissue (scar tissue that maintains a state of irritation at the root).

An endoscope enables display and inspection of any adhesions, to identify the primary cause of inflammation and intervene accordingly.

Medical literature confirms the greater effectiveness, in terms of pain relief, of this therapeutic approach, as compared with other analgesic therapies.

For the procedure to be performed successfully, an endoscope should ideally permit display of the target area and, at the same time, allow the operator a comfortable margin in which to adjust and orient the endoscopy tool with respect to the tissues for analysis and/or treatment.

Obviously, an endoscope must be compatible with the degree of sensitivity of the target area. This is particularly crucial in the case of spinal endoscopy, in which the operator is called upon to work in a space - the peridural space - of minute size (roughly a few millimetres in an adult male) bounded by highly sensitive tissue.

The peridural space, in fact, is substantially defined by the spinal foramen between the bone walls of the vertebrae and the dura mater covering the spinal cord. The spinal foramen mostly contains adipose tissue, but is also coursed by numerous blood and lymph vessels, and contains the dura-arachnoid projections surrounding the roots of spinal nerves. A high degree of precision and reliability is therefore demanded of the endoscope, combined with strict safety features in terms, for example, of sterile component parts; and effective monitoring of any interaction between the component parts of the endoscope and the target area tissue is also crucial.

US5396880 describes a spinal endoscope comprising a catheter, in turn comprising a lumen into which slides a bundle of first and second optical fibres, separate and independent of the endoscope. The bundle is smaller in diameter than the lumen, the first optical fibres are connectable to a light source to illuminate the endoscope target area, and the second optical fibres are connectable to an image-acquisition device to real-time display the target area. The spinal endoscope described also comprises deflecting means, separate from the optical fibre bundle, for deflecting the distal end of the catheter; and irrigation means for feeding irrigation fluid along the catheter to the distal end.

The optical fibre bundle must be sterilized prior to use, and is then slid inside the lumen of the catheter to position the display end at the distal end of the catheter, which in use is inserted inside the patient's body.

The position of the optical fibre bundle inside the catheter must therefore be adjusted precisely each time with respect to the open end of the catheter. More specifically, it is recommended to keep the optical fibre bundle in a withdrawn position as the distal end of the catheter is inserted inside the peridural space, and to adjust its position later. This operation must therefore be controlled radiographically, using radiopaque markers on the end of the optical fibre bundle and the open end of the catheter.

This solution has the drawback of having to perform a delicate, precise adjustment after the endoscopy has actually commenced, thus prolonging anaesthetization of the patient, and also exposure of the patient to ionizing radiation to ensure correct location of the optical fibre bundle.

Moreover, there is always the risk, due to human or technical error, of the display end of the optical fibre bundle being slid through the open end of the catheter and directly into the peridural space. Given the minute size of the endoscopy target area, such an error, though geometrically insignificant, may obviously result in serious damage to highly sensitive tissue.

The US5396880 solution also requires sterilization of the optical fibre bundle prior to use, which, besides costing time and money, has the drawback of impairing performance, i.e. seriously reducing the number of operating cycles, of the optical fibres in the bundle.

US6146355 describes a manoeuvrable catheter, i.e. with a flexible end and user-controlled deflecting means, which comprises a lumen, into which is slid an optical fibre bundle substantially similar to that of US5396880, and is fitted on its distal end with a lens for focusing and transmitting the image to the optical fibre bundle. In this case, the lumen is closed by the lens, so the optical fibre bundle can be inserted until it comes to rest against the face of the lens facing inwards of the lumen.

Though the lens acts as a stop when inserting the optical fibre bundle, it may pose other adjustment problems to determine the best target area illumination and display position of the end of the bundle with respect to the lens.

Moreover, in the event, for example, of a manufacturing defect, or of improper adjustment resulting in the optical fibre bundle exerting too much pressure on the lens, there is always the possibility of the lens detaching from the catheter directly into the target area, thus endangering and possibly subjecting the patient to serious complications.

A need is therefore felt for an endoscope, in particular for spinal endoscopy procedures, which ensures precise, reliable visual, and preferably also operating, access to the target area, while at the same time simplifying adjustment of the endoscopy tool to speed up diagnosis/therapy and reduce patient exposure time to ionizing radiation.

Demand also exists for a patient-safe endoscope in terms of hygiene and monitoring interaction between the endoscope and target tissue.

It is an object of the present invention to provide an endoscope designed to provide a simple, low-cost solution to at least one of the above demands.

According to the present invention, there is provided an endoscope as claimed in Claim 1.

The invention also relates to an endoscopy system as claimed in Claim 11.

A preferred, non-limiting embodiment of the present invention will be described by way of example with reference to the accompanying drawings, in which :
Figure 1 shows a schematic of an endoscopy system comprising an endoscope in accordance with the present invention;

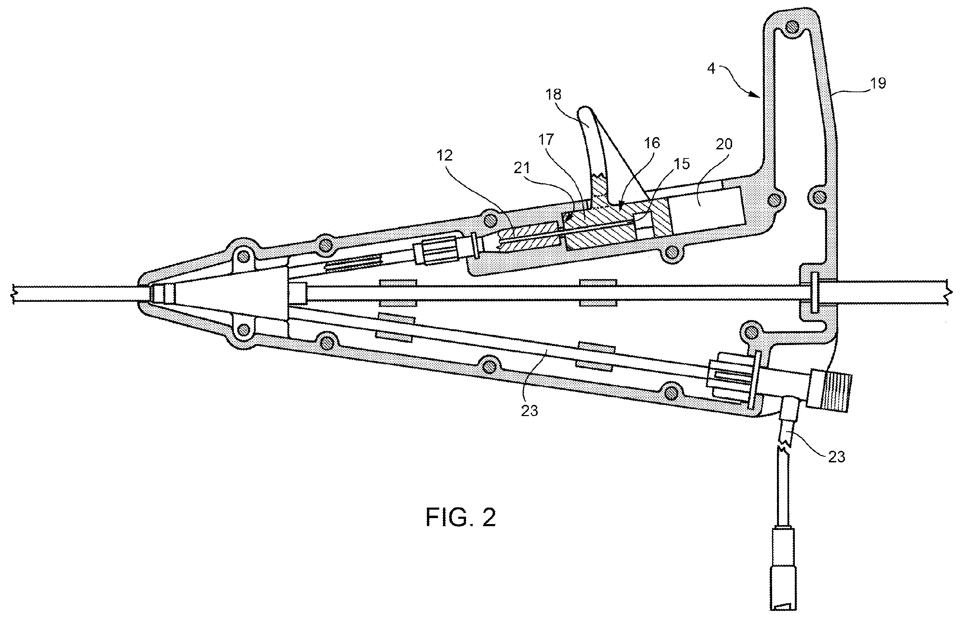

Figure 2 shows a schematic section of the handgrip of the Figure 1 endoscope;
Figures 3A and 3B show a plan and side view respectively of the Figure 2 handgrip;
Figure 4A shows a larger-scale section along line IV-IV in Figure 1;
Figure 4B shows a section along line IVB-IVB in Figure 4A;
Figure 5A shows a larger-scale view of the distal end of the Figure 1 endoscope;
Figure 5B shows a section along line VB-VB in Figure 5A.

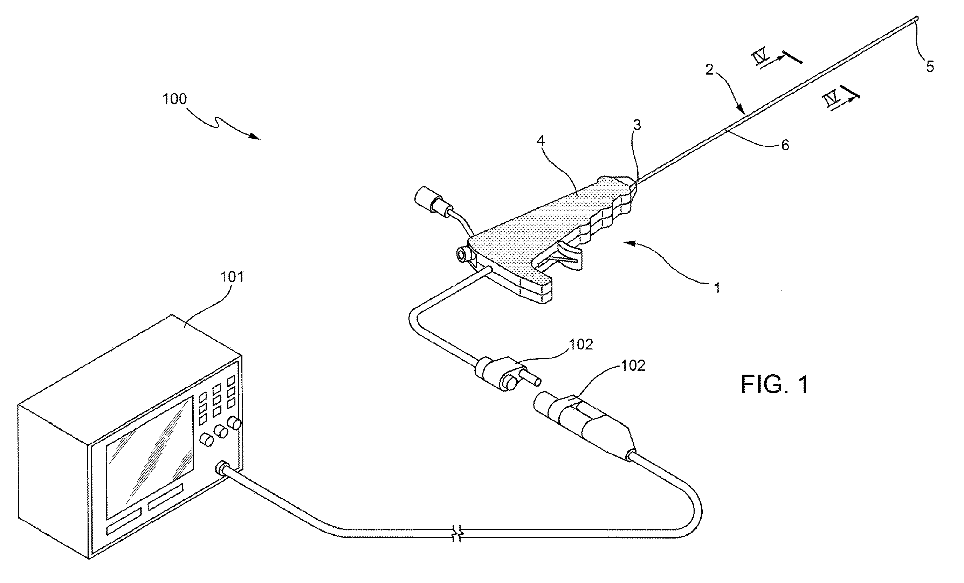

Number 100 in Figure 1 indicates as a whole an endoscopy system comprising an endoscope 1 in accordance with the present invention.

In addition to endoscope 1, endoscopy system 100 comprises a display and monitoring unit 101, to which endoscope 1 is connected by connecting means 102.

Endoscope 1 comprises a catheter 2 fixed by its proximal end 3 to a handgrip 4.

Catheter 2 is flexible at least at its distal end 5, which is insertable hypodermically into a body cavity, e.g. the peridural space, at an endoscopy target area.

Catheter 2 comprises a body 6 preferably made, e.g. extruded, from medical-suitable plastic material, such as polyurethane. Body 6 has an outside diameter of a few millimetres, typically less than 3.5 mm, for easy manoeuvring inside small target areas, such as the peridural canal, and to minimize the invasiveness of the endoscopy procedure.

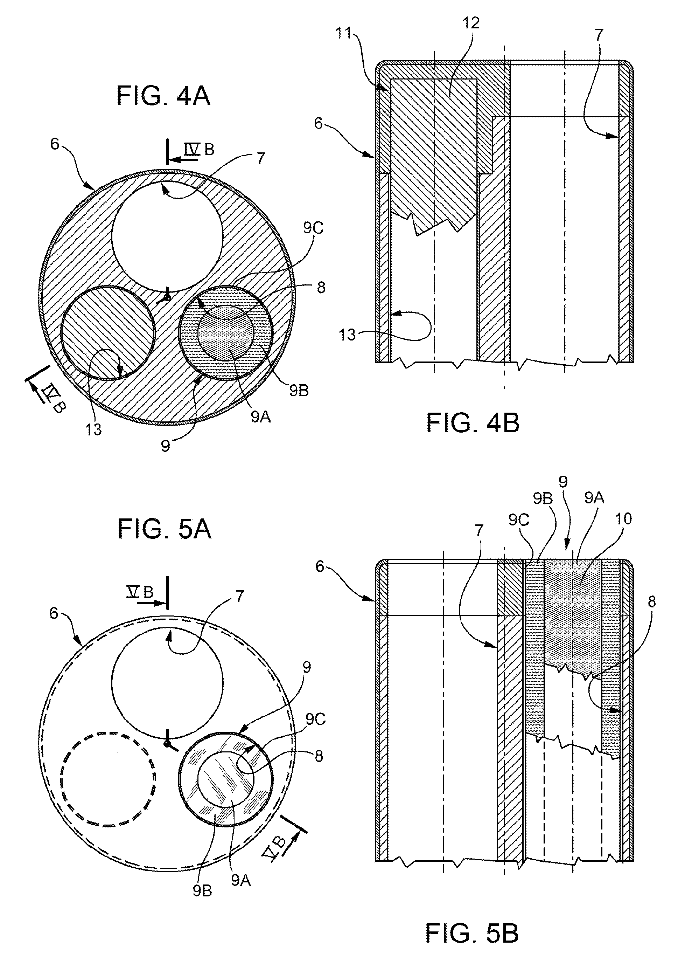

Body 6 of catheter 2 (Figures 4A-B and 5A-B) defines an operating lumen 7 and an optical lumen 8, both extending axially along body 6.

More specifically, operating lumen 7 is a channel open at both ends of body 6 and therefore connecting the outside environment fluidically to the target area, e.g. to locally administer drugs, etc.

Catheter 2 also comprises an optical fibre bundle 9 housed inside optical lumen 8 and comprising a first number 9A of illumination fibres, and a second number 9B of display fibres. The first number 9A of illumination fibres typically forms the core of bundle 9, which is at least partly surrounded by the second number 9B of display fibres positioned substantially radially out fist number 9A of fibres.

Optical fibre bundle 9 preferably also comprises a substantially tubular protective sheath 9C enclosing the fibres.

Protective sheath 9C is particularly advantageous during the manufacture of endoscope 1. Catheter body 6, in fact, may conveniently be extruded directly onto bundle 9 to ensure the outside diameter of bundle 9 substantially matches the inside diameter of optical lumen 8.

One end of optical fibre bundle 9 is advantageously fixed integrally to the distal end 5 of catheter 2 (Figure 5B) to permit direct visual access to the endoscopy target area.

The distal end 10 of bundle 9 is preferably substantially flush with distal end 5 of the catheter, so as to face directly inside the endoscopy target area, i.e. with nothing between distal end 10 of optical fibre bundle 9 and the target area; and, by virtue of the construction of catheter 2, optical fibre bundle 9 is located in the ideal endoscopy position.

Optical fibre bundle 9 is preferably 0.9 to 2.0 mm in diameter, and the display bundle has a diameter of 0.7 to 1.8 mm, with a resolution of roughly 6000-12000 pixels.

Endoscope 1 also comprises deflecting means 11 for flexing the distal end of catheter 2 with respect to its longitudinal axis.

In the example shown, deflecting means 11 comprise a metal wire 12 housed partly inside a further lumen 13 extending longitudinally inside catheter 2. More specifically, metal wire 12 has a distal end 14 and a proximal end 15. A first portion of metal wire 12, adjacent to proximal end 15, is located outside catheter 2 for connection to handgrip 4. And at the opposite end, metal wire 12 is fixed by distal end 14 to distal end 5 of catheter 2.

The proximal end 15 of metal wire 12 is advantageously fixed integrally to a fastening body 16 comprising a substantially cylindrical main portion 17, and a trigger portion 18 projecting radially from main portion 17.

In the example shown, handgrip 4 comprises a casing 19 defined, for example, by two substantially triangular half-shells molded from plastic material. Casing 19 defines a cylindrical cavity 20 of substantially the same diameter as fastening body 16, apart from appropriate tolerances.

Fastening body 16 partly engages, and is movable axially along the inside of, cavity 20. More specifically, cavity 20 of handgrip 4 has an opening, through which trigger portion 18 projects outwards of casing 19 for operation by the user.

The distal end of the catheter is thus integral with fastening body 16, so translation of fastening body 16 inside cavity 20 causes distal end 5 of catheter 2 to flex. More specifically, fastening body 16 is movable between a rest position, in which it rests against a front stop shoulder 21 of cavity 20, and a position corresponding to maximum flexure of the distal end of catheter 2, which depends on the flexibility of the material from which catheter 2 made, the tension of metal wire 12, and the length of cavity 20 and more specifically of the opening in the cavity.

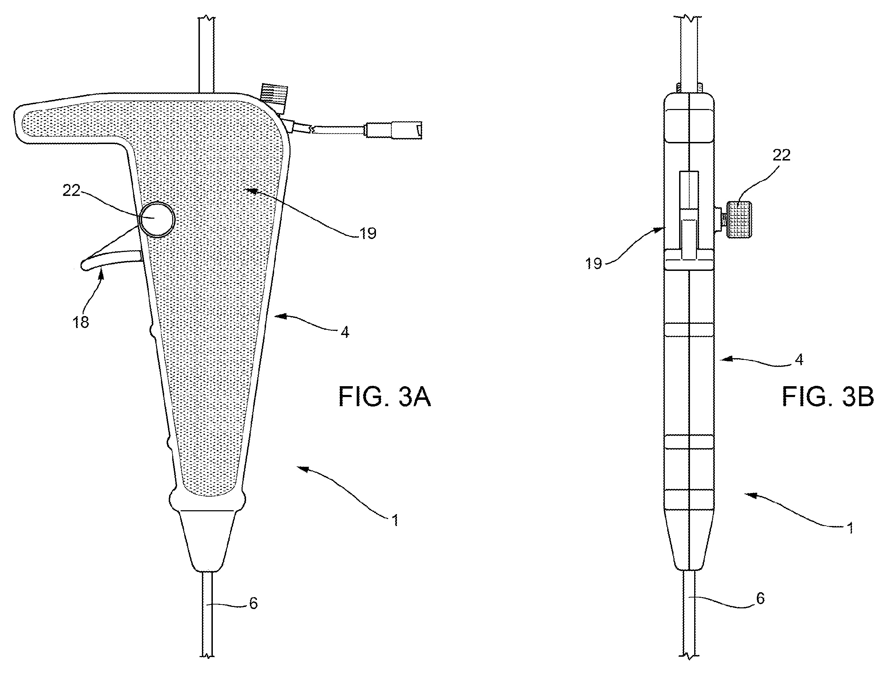

Handgrip 4 (Figures 3A-B) also comprises stop means 22 for securing fastening body 16 releasably in a given position inside cavity 20, and defined, for example, by a thumbscrew.

In an alternative embodiment not shown, endoscope 1 may comprise second deflecting means, e.g. identical to and symmetrical, for the sake of simplicity, with those described above. In other words, endoscope 1 may comprise a second metal wire housed in a respective further lumen in the catheter, and operated by translation of a respective fastening body housed slidably inside the handgrip, so as to flex the distal end of the catheter in opposite directions with respect to the longitudinal axis of the catheter.

In the example shown (Figures 4A-B and A-B), operating lumen 7 communicates fluidically with the outside along a tube portion 23 housed in and fixed releasably to at least one of the half-shells of casing 19.

Handgrip 4 also comprises means for connecting optical fibre bundle 9 to display and monitoring unit 101, preferably by connecting means 102.

Optical fibre bundle 9 is also advantageously fixed releasably to at least one of the half-shells of casing 19.

In actual use, deflecting means 12 are operated using trigger portion 18 of fastening body 16, which, as it slides along cavity 20, exerts pull on metal wire 12 to flex the distal end of catheter 2, and can be stopped in a given position by means of stop means 22 to set the distal end of the catheter to a given flexed position.

The advantages of the endoscope according to the present invention will be clear from the above description.

In particular, optical fibre bundle 9 is set beforehand to the best position and needs no further adjustment with respect to the distal end 5 of catheter 2, thus substantially eliminating any risk of it being positioned wrongly, while at the same time shortening the endoscopy procedure and reducing patient exposure time to ionizing radiation.

More specifically, not being subject to any adjustment, there is virtually no risk of optical fibre bundle 9 overshooting the distal end 5 of catheter 2 and penetrating directly into the endoscopy target area.

In addition, direct visual access to the target area, i.e. with no lens on the distal end 5 of catheter 2, provides for more easily controllable image quality, by being independent of the position of the end of bundle 9 with respect to the lens.

Moreover, the whole of endoscope 1 being disposable, there is no need to sterilize optical fibre bundle 9 before each use. In other words, each endoscopy procedure is performed using a new, completely sterile endoscope 1, thus improving hygiene conditions and reducing risk to the patent.

Clearly, changes may be made to the system as described and illustrated herein without, however, departing from the scope of the independent Claims.

## Claims

1. An endoscope (1) comprising :
a catheter (2) comprising a flexible body (6) defining, along its longitudinal axis, at least an operating lumen (7) and an optical lumen (8); the body (6) comprising a distal end (5) insertable into an endoscopy target area; and
an optical fibre bundle (9) housed in said optical lumen (8);
the endoscope being **characterized in that** said optical fibre bundle (9) is fixed firmly at one end (11) to said distal end (5) of said body (6) to permit direct visual access to said target area.

2. An endoscope as claimed in Claim 1, wherein said end (11) of said optical fibre bundle (9) is substantially flush with said distal end (5) of the body (6), so as to face directly inside said target area in use.

3. An endoscope as claimed in Claim 1 or 2, wherein said optical fibre bundle (9) comprises a first number (9A) of illumination fibres and a second number (9B) of display fibres.

4. An endoscope as claimed in any one of Claims 1 to 3, wherein said optical fibre bundle (9) comprises a tubular protective sheath (9C) enclosing the fibres.

5. An endoscope as claimed in any one of Claims 1 to 4, and comprising deflecting means (11) for flexing said distal end (5) with respect to said longitudinal axis.

6. An endoscope as claimed in Claim 5, wherein said body (6) defines a longitudinal further lumen (13); said deflecting means (11) comprising a metal wire (12) housed in said further lumen (13) and fixed at its distal end to said distal end (5) of said body (6).

7. An endoscope as claimed in Claim 6, and comprising a handgrip (4); said metal wire (12) being fixed at its proximal end to said handgrip (4).

8. An endoscope as claimed in Claim 7, wherein said metal wire (12) is fixed to a fastening body (16) movable inside a cavity (20) in said handgrip (4).

9. An endoscope as claimed in Claim 8, wherein said fastening body (16) comprises a trigger portion (18) user-operated to translate said fastening body (16) inside said cavity (20).

10. An endoscope as claimed in any one of Claims 7 to 9, and comprising stop means (22) for releasably locking said metal wire (12) in a predetermined flexed position.

11. An endoscopy system (100) comprising an endoscope as claimed in any one of Claims 1 to 10, and a display and monitoring unit (101).
